# EUROPEAN PATENT APPLICATION

(11) **EP 4 302 804 A1**
(43) Date of publication of application: **10.01.2024**
(21) Application number: 21928676.2
(22) Date of filing: 12.05.2021
(51) Int. Cl.: A61M 5/178, A61B 6/00

(54) **PORTABLE CO2 GAS FILLER**

(30) Priority: 01.03.2021 CN 202110223553
(71) Applicant: Beijing Advanced Medical Technologies, Ltd., Inc., Beijing 102609 (CN)
(72) Inventor: ZHAO, Hugh Qinghong, Pleasanton, California 94588 (US); NIKANOROV, Alexander, Beijing 102609 (CN); ZHAO, Qinghua, Beijing 102609 (CN); LIU, Qing, Beijing 102609 (CN); WANG, Helong, Beijing 102609 (CN); DU, Hong, Beijing 102609 (CN); LU, Lele, Beijing 102609 (CN)
(74) Representative: Kurig, Thomas
(86) International application number: PCT/CN2021/093273
(87) International publication number: WO 2022/183595

(57) **Abstract**

A portable CO₂ gas injector, comprising an injecting tube (01). One end of the injecting tube (01) is communicated with an inlet and outlet tube (02), and the end of the inlet and outlet tube (02) distant from the injecting tube (01) is communicated with an injecting valve (03); the end of the injecting valve (03) distant from the inlet and outlet tube (02) is communicated with a gas injecting tube (04); the end of the gas injecting tube (04) distant from the injecting valve (03) is communicated with a communicating member (051), and one side of the communicating member (051) is communicated with a gas cylinder (052); a piston (011) matching the injecting tube (01) is arranged in the injecting tube (01), and a spring (06) for driving the piston (011) to move and an adjusting assembly (07) for adjusting the gas storage volume are arranged on the side of the piston (011) distant from the inlet and outlet tube (02); when the injection amount needs to be adjusted, the adjusting assembly (07) is used to adjust the volume available for gas storage in the injecting tube (01), thereby achieving the effects of performing quantitative control on the injection amount of carbon dioxide and then relieving discomfort of patients.

## Description

### Technical Field

This application relates to the field of injection technology, especially a portable carbon dioxide gas injector.

### Background Technology

As carbon dioxide can naturally exist in the human blood vessels, is biocompatible, soluble in blood, can be eliminated through exhalation from the lungs, and is non-toxic to the liver and kidneys, so it is often used as a contrast agent for vascular angiography. In related techniques, when carbon dioxide needs to be injected into the blood vessels, the operator first uses a manual syringe to extract carbon dioxide from a cylinder assembly into the manual syringe, and then injects carbon dioxide into an external injection device that is in connection with the patient's vessels, and carbon dioxide enters the vessels through the external injection device to achieve vascular angiography.

Regarding the above related technical solutions, the inventor found that different vessels can withstand different volumes of carbon dioxide. Because the carbon dioxide inside the manual syringe is highly compressed, when injecting carbon dioxide into different parts of the vessels, the operator needs to exert a lot of force, which makes it difficult to accurately control the injection volume and may cause discomfort to the patient.

### Brief Summary

To be able to quantitatively control the injection volume of carbon dioxide and thus alleviate patient discomfort, the present application provides a portable carbon dioxide gas injector.

The portable carbon dioxide gas injector provided by the present application adopts the following technical solution:
The portable carbon dioxide gas injector includes an injecting tube, one end of which is connected with a passage tube. The end away from the injecting tube, of the passage tube is connected with an injecting valve. An input assembly is connected with the end away from the injecting tube, of the injecting valve. The input assembly is connected to a connector at the end away from the pass valve, and a gas cylinder is connected to the other side of the connector. The injecting tube is provided with a piston suitable for the injecting tube, and a spring for driving the piston to move is provided on the side away from the passage tube, of the piston, as well as a regulating assembly for regulating the gas storage volume.

By using the above technical solution, when the patient needs to undergo angiography, the operator first uses the connector and input assembly to smoothly discharge the carbon dioxide from the gas cylinder into the injecting tube through the injecting valve and passage tube. When it is needed to inject carbon dioxide into the patient's vessels, the spring drives the piston to inject carbon dioxide from the injecting tube into the patient's vessels. When it is necessary to regulate the injection volume, the regulating assembly is used to regulate the gas storage volume in the injecting tube, and then carbon dioxide in the injecting tube is injected into the patient's vessels. The regulating assembly is able to regulate the gas storage volume in the injecting tube, achieving precise quantitative control of the injection volume of carbon dioxide, thereby reducing patient discomfort. The piston separates the carbon dioxide from the spring and the regulating assembly, reducing the possibility of other gases seeping into the carbon dioxide and ensuring the purity of the carbon dioxide. The connector, input assembly, injecting valve, and passage tube facilitate the smooth discharge of carbon dioxide into the injecting tube, improving the convenience of operation.

Preferably, the driving assembly includes a spring, one end of which is fixedly connected to the piston, and the other end of which is fixedly connected to the inner wall of the injecting tube.

By using the above preferred technical solution, when the carbon dioxide in the cylinder assembly enters the injecting tube through the connector, input assembly, injecting valve and the passage tube, the spring is compressed by the carbon dioxide, and when it is necessary to inject carbon dioxide into the patient's vessels, the injecting valve is switched to output state, and the spring is able to drive the piston towards the direction close to the passage tube, thereby achieving discharge of the gas in the injecting tube. The spring is set to drive the piston to discharge the carbon dioxide from the injecting tube to the outside world, reducing the operator's effort in injecting carbon dioxide and lowering labor intensity, while increasing operational efficiency and convenience.

Preferably, the regulating assembly includes a regulating rod that passes through the injecting tube and is threadedly connected to the injecting tube. A gasket is fixedly mounted at one end close to the piston, of the regulating rod.

By using the above technical solution, when carbon dioxide gas enters the injecting tube through the connector, input assembly, injecting valve and the passage tube, the carbon dioxide gas pushes the piston towards the direction away from the passage tube. When the end surface of the piston touches the end surface of the regulating rod, injection can be stopped. When it is needed to inject different volumes of carbon dioxide into different vessels, the regulating rod can be rotated to regulate the position of the contacting surface of the regulating rod and the piston, thereby achieving adjustment of the gas storage volume in the injecting tube. The regulating rod is set to be able to regulate the gas storage volume in the injecting tube, facilitating the injection of different volumes of carbon dioxide, thereby reducing the possibility of discomfort to the patient caused by excessive injection of carbon dioxide, improving the versatility and convenience of use. The gasket provides cushioning and shock absorption to the piston, reducing the possibility of injury to the piston caused by contact with the regulating rod during adjustment, and improving the safety of the piston.

Preferably, a regulating knob is provided on the connector to regulate the gas pressure input into the injecting tube.

By using the above technical solution, when it is needed to inject carbon dioxide into the injecting tube, the regulating knob is turned to allow the carbon dioxide in the gas cylinder to be discharged into the injecting tube through the connector and input assembly. The connector is set to make it easier to discharge the carbon dioxide from the gas cylinder into the injecting tube, improving the convenience of use. The regulating knob allows the operator to control the flow of carbon dioxide into the injecting tube, enhancing the convenience of operation. Additionally, the regulating knob can be used to regulate the pressure of carbon dioxide in the input assembly, increasing the versatility and convenience of use.

Preferably, the connector includes a pressure gauge to display the gas pressure inside the gas cylinder.

With above configuration, the gas pressure inside the gas cylinder can be observed by operators using the pressure gauge, allowing them to determine if a replacement cylinder is needed.

Preferably, a filter is provided on the input assembly to filter the gas passing into the injecting tube.

With above configuration, the carbon dioxide gas passed through the filter is secondarily filtered, enhancing its purity and reducing the possibility of discomfort to patients.

Preferably, the regulating assembly includes a moving plate that is adapted to the inner wall of the injecting tube. One end of the spring is fixedly connected to the piston, while the other end of the spring is fixedly connected to the moving plate. The end face close to the piston, of the moving plate is connected to a docking block, while the end face away from the piston, of the moving plate is connected to a moving rod. A fixing component is provided on the injecting tube to fix the moving rod.

By using the above technical solution, when injecting certain amounts of carbon dioxide into the patient's vessels, the moving rod is first secured using the fixing component. Due to the fixed lengths of the docking block and the moving rod, after discharging carbon dioxide into the injecting tube, the docking block will contact the piston, ensuring the volume of carbon dioxide within the injecting tube remains constant. The combination of the moving rod, docking block, and fixing component achieves the adjustment of gas storage volume within the injecting tube, facilitating the injection of carbon dioxide of different volumes into the human blood vessels and reducing the likelihood of discomfort caused by excessive injection.

Preferably, the fixing component includes a fixing rod perpendicular to the axis of the moving rod, with multiple fixing holes provided on the moving rod along its extension direction. One side of the fixing rod is equipped with a fixing box that is fixedly connected to the injecting tube, and the fixing box has an open end facing the moving rod. A moving block that is adapted to the fixing box is provided inside the fixing box, and the moving block is slidably connected to the fixing box. The fixing rod passes through the moving block and is fixedly connected to it.

By using above technical solution, when it is needed to regulate the gas storage volume within the injecting tube, first move the fixing rod away from the moving rod. Next, move the moving rod to a certain distance, and insert the fixing rod into the corresponding fixing hole. Subsequently, inject highly compressed carbon dioxide into the injecting tube, causing the piston to contact the docking block, eventually storing the predetermined amount of carbon dioxide within the injecting tube. The combination of the fixing rod and the fixing box can fix the fixing rod, thereby enabling the fixing rod to fix the moving rod, reducing the possibility of the moving rod moving due to the impact of highly compressed carbon dioxide, and improving the stability of the moving rod.

Preferably, the fixing rod is sleeved with a limiting spring, one side of which is fixedly connected to the inner wall away from the moving rod, of the fixing box, and the other side of which is fixedly connected to the moving block.

By using above technical solutions, when it is needed to regulate the gas storage volume within the injecting tube, first move the fixing rod away from the moving rod, and the limiting spring is compressed by the moving block. Next, move the moving rod to a certain distance, and release the fixing rod once the moving rod is in desired position. The fixing rod is inserted into the fixing holes under the force of the limiting spring, thereby achieving the fixing of the moving rod. The limiting spring is set to facilitate the automatic reset of the fixing rod, as well as secure the fixing rod after the fixing rod is inserted in the fixing holes, thereby reducing the possibility of the fixing rod popping out of the fixing holes due to the impact force of carbon dioxide, and improving the stability of the fixing rod when fixing the moving rod.

Preferably, the gas cylinder is detachably connected to the connector. One end of the injecting valve is connected to a Luer connector, and the other end away from the injecting valve, of the Luer connector is provided with a check valve. The injecting tube is covered with a protective cover on its outer side.

By using above technical solutions, when the carbon dioxide gas in the gas cylinder is depleted, the detachable connection between the gas cylinder and connector facilitates easy replacement with a new cylinder, and then a new gas cylinder is used to provide carbon dioxide into the injecting tube. The protective sleeve is set to facilitate gripping of the injecting tube for the operator, as well as protect the injecting tube, reducing the possibility of damage caused by external impact. Due to the fact that the Luer connector is a non-leakage connector for trace fluids, when the carbon dioxide gas in the injecting tube is injected into the patient's vessels, the Luer joint reduces the possibility of ambient air infiltration into the carbon dioxide that is to be injected into the patient's vessels, ensuring the purity of the carbon dioxide. The check valve is set to ensure the carbon dioxide to only be discharged outwardly, further reducing the possibility of external gas entering the Luer connector and the injecting valve.

In summary, the present application provides the following technical effects:
1. By setting a regulating assembly, the volume of gas that can be stored in the injecting tube is adjusted through the regulating assembly when needed. The regulating assembly can regulate the gas storage volume of the injecting tube, which achieves quantitative control of the injection volume of carbon dioxide, thereby alleviating discomfort in patients.
2. When it is needed to inject certain amounts of carbon dioxide into the patient's vessels, first utilize the fixing component to fix the moving rod. Due to the fixed length of the docking block and the moving rod, the piston will contact the docking block after carbon dioxide is discharged into the injecting tube, thereby limiting the carbon dioxide in the injecting tube to a certain volume, reducing the possibility of patient discomfort caused by excessive injection.
3. When it is needed to regulate the gas storage volume within the injecting tube, first move the fixing rod away from the moving rod. Secondly, move the moving rod to a certain distance. Then, insert the fixing rod into the corresponding fixing hole. Subsequently, highly compressed carbon dioxide is injected into the injecting tube, causing the piston to contact the docking block, eventually storing the predetermined amount of carbon dioxide within the injecting tube. This reduces the possibility of the moving rod popping out of the fixing holes due to the impact force of carbon dioxide, and improving the stability of the fixing rod when fixing the moving rod.

### Drawings

Fig. 1 is a structural schematic diagram of the injector with in the Embodiment 1 of the present application.
Fig. 2 is a structural schematic diagram of the injector with a protective cover hidden in the Embodiment 1 of the present application.
Fig. 3 is a sectional view highlighting the driving assembly and the regulating assembly in the Embodiment 1 of the present application.
Fig. 4 is a structural schematic diagram of the injector in the Embodiment 2 of the present application.
Fig. 5 is a sectional view highlighting the driving assembly and the regulating assembly in the Embodiment 2 of the present application.
Fig. 6 is a partial sectional view highlighting the fixing component in the Embodiment 2 of the present application.
Fig. 7 is an enlarged schematic diagram of location A in Fig. 6.
Fig. 8 is an angiographic effect diagram of carbon dioxide injected into the femoral artery of a human body.
Fig. 9 is an angiographic effect diagram of carbon dioxide injected into the aorta of a human body.

In the drawings, 01 refers to the injecting tube; 011 refers to the piston; 012 refers to the sealing ring; 013 refers to the scale line; 02 refers to the passage tube; 03 refers to the injecting valve; 031 refers to the rotary handle; 04 refers to the input assembly; 041 refers to the first connecting tube; 042 refers to the second connecting tube; 05 refers to the cylinder assembly; 051 refers to the connector; 0511 refers to the pressure gauge; 052 refers to the gas cylinder; 053 refers to the regulating knob; 06 refers to the spring; 07 refers to the regulating assembly; 071 refers to the regulating rod; 072 refers to the regulating cap; 073 refers to the gasket; 074 refers to the moving plate; 075 refers to the docking block; 076 refers to the moving rod; 0761 refers to the fixing hole; 077 refers to the handle; 08 refers to the filter; 09 refers to the Luer connector; 10 refers to the check valve; 11 refers to the protective cover; 111 refers to the observation groove; 12 refers to the fixing component; 121 refers to the fixing rod; 122 refers to the fixing box; 123 refers to the moving block; 124 refers to the limiting spring; and 125 refers to the pull ring.

### Detailed Description of Embodiments

This application is further explained in detail in conjunction with the following attached drawings_{∘}

### Embodiment 1:

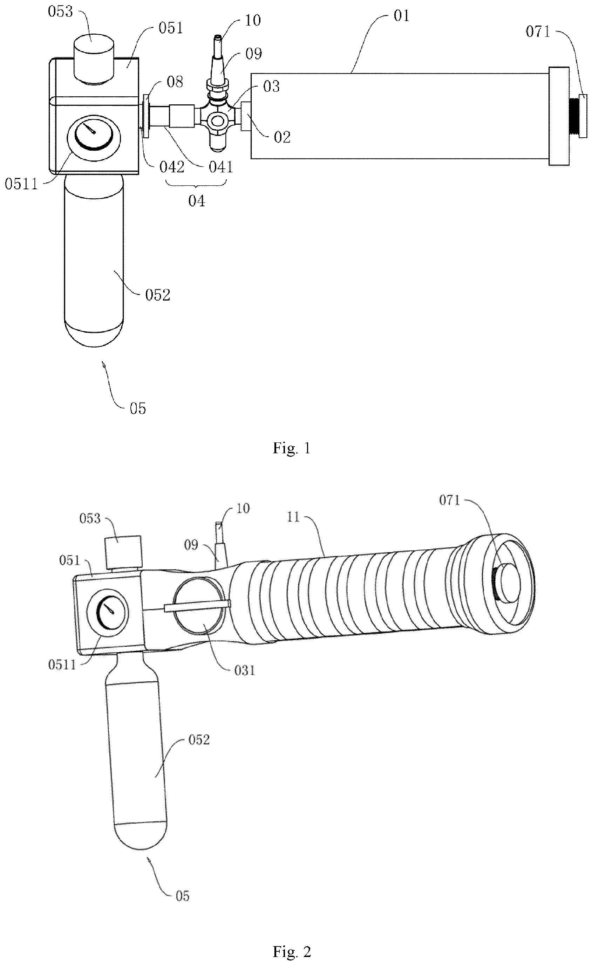

Referring to Figures 1 and 2, the present application provides a portable carbon dioxide gas injector, including a horizontally arranged injecting tube 01 that is approximately a cylinder. One end of the injecting tube is integrally formed with an expansion head, and the diameter of the expansion head is greater than that of the injecting tube 01. The end away from the expansion head, of injecting tube 01 is connected to a passage tube 02 which is coaxial with the injecting tube 01, and the other end away from the injecting tube 01, of the passage tube 02, is equipped with an injecting valve 03, which is a three-way valve. A rotary handle 031 is fixed on the injecting valve 03. The end away from the injecting tube 01, of the injecting valve 03 is connected to an input assembly 04, and a cylinder assembly 05 is arranged at the end away from the injecting valve 03, of the input assembly 04. External injection devices connected to vessels of the patient are arranged on one side of the injecting valve 03.

When the patient needs to undergo angiography, the operator first switches the injecting valve 03 to make the input assembly 04 in communication with the passage tube 02. Then, the cylinder assembly 05 and the input assembly 04 are used to input carbon dioxide into the injecting tube 01. After the input is completed, the injecting valve 03 is switched to make the passage tube 02 in communication with the external injection devices. The carbon dioxide in the injecting tube 01 is then injected into the patient's vessels, allowing the patient to undergo angiography. The setting of the injecting valve 03 integrates the cylinder assembly 05, the injecting tube 01, and the external injection device, reducing the possibility of external gas permeating into the carbon dioxide gas, ensuring the purity of the carbon dioxide, and reducing the possibility of discomfort in patients caused by other gases mixed with the carbon dioxide. The setting of the injecting valve 03 makes it easy to inject carbon dioxide into the patient's vessels by only switching the injecting valve 03, improving the ease of use. The rotary handle 031 can only be operated in two states, which are making the input assembly 04 and the passage tube 02 in communication and making the passage tube 02 and the external injection devices in communication. It makes the switching of the rotary handle 031 a foolproof operation with only two operational states, reducing the possibility of incorrect operation by the operator

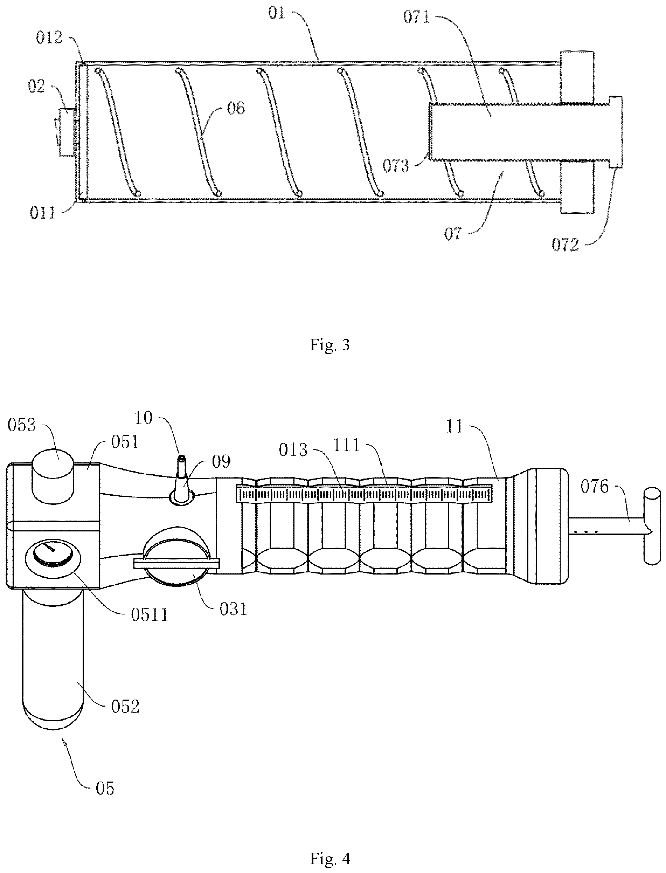

Referring to Fig. 3, a piston 011, coaxial with the injecting tube 01, is arranged inside the injecting tube 01. The piston 011 is adapted to the injecting tube 01. A sealing ring 012 is set on the periphery of the piston 011. On the side of the piston 011 away from the passage tube 02, a spring 06 is provided to drive the piston 011 to move. One end of the spring 06 is fixedly connected to the end face of the piston 011 away from the passage tube 02, and the other end of the spring 06 is fixedly connected to the inner wall of the injecting tube 01.

When injecting carbon dioxide into the patient's vessels, the injecting valve 03 is switched to allow the carbon dioxide in the cylinder assembly 05 to enter the injecting tube 01. As the carbon dioxide inside the injecting tube 01 is highly compressed gas, the piston 011 and the spring 06 move towards the end away from the passage tube 02 under the pressure of the carbon dioxide. After a certain amount of carbon dioxide is injected into the injecting tube 01, the injecting valve 03 is switched to make the passage tube 02 in communication with the external injection devices. The carbon dioxide is pushed into the external injection devices by the spring 06. The piston 011 separates the carbon dioxide from the spring 06 and regulating assembly 07, reducing the possibility of other gases permeating into the carbon dioxide gas, ensuring the purity of the carbon dioxide. The sealing ring 012 further improves the sealing of the space storing the carbon dioxide gas. The spring 06 facilitates the discharge of carbon dioxide from the injecting tube 01, eliminating the need for the operator to exert much force to inject carbon dioxide into the patient's vessels, reducing labor intensity, and improving operational efficiency and convenience.

Referring to Fig. 3, the regulating assembly 07 is provided on the side away from the passage tube 02, of the piston 011 to regulate the gas storage volume. The regulating assembly 07 includes a regulating rod 071 coaxial with the injecting tube 01. The regulating rod 071 is threadedly connected to the injecting tube 01, and there is a threaded hole on the injecting tube 01 for the regulating rod 071 to rotate. The end away from the piston 011, of the regulating rod 071 is fixedly connected to a regulating cap 072, and the other end near the piston 011, of the regulating rod 071is fixedly connected to a gasket 073.

When the carbon dioxide enters the injecting tube 01 through the injecting valve 03 and the passage tube 02, the carbon dioxide pushes the piston 011 to move towards the end away from the passage tube 02. The piston moves until the end face of the piston 011 is in contact with the end face of the regulating rod 071. When the gas storage volume inside the injecting tube 01 needs to be adjusted, the operator rotates the regulating rod 071 along the axis of the injecting tube 01 to change the position of the contact surface of the piston 011 and the regulating rod 071 to realize the adjustment of gas storage volume in injecting tube 01. The regulating rod 071 realizes the adjustment of the gas storage volume inside the injecting tube 01, facilitating the injection of carbon dioxide of different volumes into the human vessels. This reduces the possibility of discomfort in patients caused by injecting too much carbon dioxide. The threaded connection between the regulating rod 071 and the injecting tube 01 reduces the possibility of the regulating rod 071 being pushed by highly compressed carbon dioxide, which facilitates the quantitative control of the injection of carbon dioxide. The regulating cap 072 facilitates the rotation of the regulating rod 071, improving ease of use. The gasket 073 provides damping and slows down the movement of the piston 011 when it contacts the regulating rod 071, reducing the possibility of the piston 011 being damaged during rapid movements, improving the safety of the piston 011, and extending its service life.

Referring to Fig. 2, the cylinder assembly 05 includes a connector 051 connected to the input assembly 04. One side of the connector 051 is connected to a gas cylinder 052, which stores highly compressed carbon dioxide. The gas cylinder 052 is detachably connected to the connector 051. The connector 051 has a regulating knob 053 for regulating the gas pressure input into the injecting tube 01. The connector 051 is provided with a pressure gauge 0511 for displaying the gas pressure inside the gas cylinder 052.

When inputting carbon dioxide into the injecting tube 01, the regulating knob 053 is rotated to allow carbon dioxide from the gas cylinder 052 to be input into the injecting tube 01 through the connector 051. The connector 051 facilitates the input of carbon dioxide into the injecting tube 01, and the regulating knob 053 allows the operator to control the pressure of carbon dioxide injected into the injecting tube 01, making the use of the injector more versatile and convenient. The pressure gauge 0511 allows the operator to observe the remaining gas pressure in the gas cylinder 052 and decide whether to replace the gas cylinder 052. With the detachable connection between the gas cylinder 052 and the connector 051, the operator can easily replace the gas cylinder 052, making the use of the system more convenient.

Referring to Fig. 2, the input assembly 04 includes a first connecting tube 041 and a second connecting tube 042 coaxial with the first connecting tube 041. A filter 08 coaxial with the first connecting tube 041 is fixed on an end surface near the second connecting tube 042. The filter 08 is designed to perform secondary cleaning by filtration on the carbon dioxide charged into the injecting tube 01, further improving the purity of the carbon dioxide and reducing the likelihood of patients experiencing discomfort symptoms.

Referring to Figs. 2 and 3, one end of the injecting valve 03 is connected to a Luer connector 09, of which the end away from the injecting valve 03 is connected to a check valve 10. When injecting carbon dioxide into the patient's vessels, the injecting valve 03 is rotated to connect the passage tube 02 to the external injection device. Then, under the action of the spring 06 and the piston 011, the carbon dioxide passes through the injecting valve 03, Luer connector 09, and check valve 10 to enter the external injection device connected to the patient's vessels, allowing blood vessels to perform angiographic imaging. The Luer connector 09 reduces the likelihood of outside gas infiltrating into the carbon dioxide injected into the patient's vessels, ensuring the purity of carbon dioxide and reducing patient discomfort. The check valve 10 only allows the carbon dioxide to discharge outwardly, further reducing the possibility of outside gas entering the injecting valve 03.

Referring to Figs. 1 and 2, a protective cover 11 is provided on the injecting tube 01, cylinder assembly 05, and input assembly 04 for protection. The protective cover 11 facilitates the operator's grip on the injecting tube 01, and protects the injecting tube 01, cylinder assembly 05, and input assembly 04 from being damaged by external impact, thereby improving their safety.

In summary, the process of this application is as follows: when carbon dioxide needs to be injected into the patient's vessels, the handling knob 031 is rotated to connect the input assembly 04 with the passage tube 02. Then, the knob 053 is rotated to input the carbon dioxide into the injecting tube 01 from the gas cylinder 052, and the spring 06 is compressed by the highly compressed carbon dioxide gas. After the inputting process is completed, when carbon dioxide needs to be injected into the patient's vessels, the handling knob 031 is rotated to connect the passage tube 02 with the external injection device, and the spring 06 forces the carbon dioxide in the injecting tube 01 to inject into the patient's vessels for angiographic imaging of vessels. When regulating the gas storage volume in the injecting tube 01, the regulating rod 071 is rotated to move along the axis of the injecting tube 01, so that the position where the piston 011 and the regulating rod 071 meets is changed to regulate the gas storage volume. When the carbon dioxide in the gas cylinder 052 is insufficient, the gas cylinder 052 can be detached from the connector 051 and replaced with a new gas cylinder 052.

### Embodiment 2:

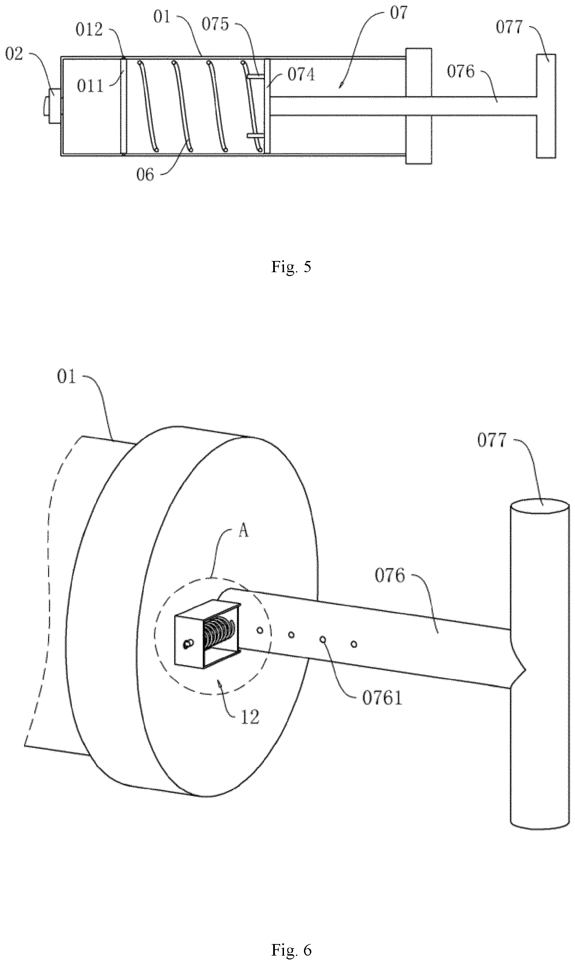

Referring to Figs. 4 and 5, the difference between Embodiment 2 and Embodiment 1 is that the regulating assembly 07 of Embodiment 2 includes a moving plate 074 coaxial with the injecting tube 01, adapted to the injecting tube 01, located at the end away from the passage tube 02, of the piston 011, with one end of the spring 06 fixedly connected to the piston 011 and the other end fixedly connected to the moving plate 074. Two horizontal docking blocks 075 are fixed on the end face of the moving plate 074 close to the piston 011, symmetrically arranged along the axis of the moving plate 074. A moving rod 076 coaxial with the injecting tube 01 is fixed on the end away from the piston 011, of the moving plate 074, and a handle 077 is fixed on the end away from the moving plate 074, of the moving rod 076. Referring to Fig. 6, a fixing component 12 is provided on the injecting tube 01 for fixing the moving rod 076, and a through hole is provided on the injecting tube 01 for the moving rod 076 to move. Scale lines 013 are provided on the side surface of the injecting tube 01, and an observation groove 111 is provided on the protective cover 11 for the operator to observe the scale lines 013.

When quantitatively injecting carbon dioxide into the patient's vessels, the moving rod 076 is fixed by using the fixing component 12. As the length of the docking block 075 and the moving rod 076 are determined, after injecting the injecting tube 01 with highly compressed carbon dioxide, the piston 011 abuts against the docking block 075 to define the amount of carbon dioxide in the injecting tube 01 quantitatively. The operator can observe the scale lines 013 corresponding to the piston 011 to determine the gas storage capacity of the injecting tube 01, achieving accurate control over the injection amount of carbon dioxide to relieve patient discomfort. The docking block 075 facilitates the quantitative control of carbon dioxide injection amount, improving the convenience of use; the moving rod 076 is combined with the fixing component 12 to regulate the gas storage volume in the injecting tube 01, making it easier for injecting tube 01 to accommodate and output different volumes of carbon dioxide into human vessels to reduce the likelihood of patient discomfort caused by overdoses; the fixing component 12 reduces the possibility of the adjustment rod 071 being pushed due to the high compression of carbon dioxide, further facilitating the quantification control of the carbon dioxide injection amount; the handle 077 facilitates the movement of the moving rod 076, improving the convenience of operation; the scale lines 013 enable the operator to observe the gas storage capacity of the injecting tube 01, improving usability, and the observation groove 111 can be used to observe the scale lines 013.

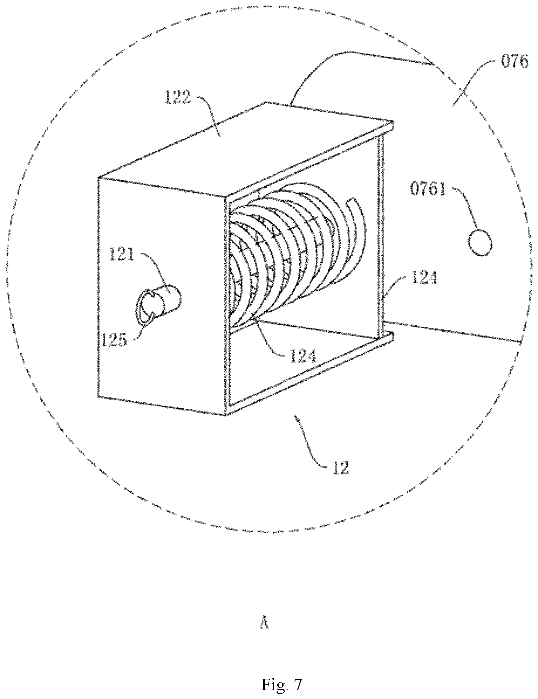

Referring to Figs. 6 and 7, the fixing component 12 includes a horizontally set fixing rod 121, with its axis perpendicular to the axis of the moving rod 076. Multiple fixing holes 0761 are uniformly provided along the extension direction of the moving rod 076 for the insertion of the fixing rod 121. The outer side of the fixing rod 121 is equipped with a hollow fixing box 122, which is fixedly connected to the injecting tube 01. The end face close to the moving rod 121, of the fixing box 122 is set to open. One end of the fixing rod 121 passes through the box wall close to the moving rod 076, of the fixing box 122, and the other end of the fixing rod 121 passes through the box wall away from the moving rod 076, of the fixing box 122. The fixing box 122 has a matching moving block 123 inside, which is slidably connected to the fixing box 122. The fixing rod 121 passes through the moving block 123 and is fixedly connected to it, and there is a through-hole on the moving block 123 for inserting the fixing rod 121.

To achieve quantitative adjustment of the gas storage volume inside the injecting tube 01, firstly move the moving rod 076 by a certain distance, then insert the fixing rod 121 into the corresponding fixing hole 0761, next input highly compressed carbon dioxide into the injecting tube 01 to make the piston 011 abut against the docking block 075 and finally store a quantitative amount of gas in the injecting tube 01. The fixing rod 121 can fix the moving rod 076, reducing the possibility of the moving rod 076 being moved by the impact of highly compressed carbon dioxide and improving the convenience of use. The fixing box 122, which cooperates with the moving block 123, can fix the fixing rod 121, enabling it to fix the moving rod 076. The multiple fixing holes 0761 facilitate the fixing of the fixing rod 121 to different positions of the moving rod 076, making it easy to regulate the gas storage volume inside the injecting tube 01 and improving the versatility and convenience of use.

Referring to Figs. 6 and 7, a limiting spring 124 is sleeved on the fixing rod 121. One end of the limiting spring 124 is fixedly connected to the inner wall away from the moving rod 076, of the fixing box 122, and the other end is fixedly connected to the moving block 123. A pull ring 125 is fixed at one end of the fixing rod 121 away from the moving rod 076.

When regulating the gas storage volume in the injecting tube 01, firstly move the fixing rod 121 away from the moving rod 076 by pulling the pull ring 125, compressing the limiting spring 124 with the action of the moving block 123, then move the moving rod 076 along the extension direction of the injecting tube 01 to the desired position, and finally release the pull ring 125 to enable the limiting spring 124 to drive the fixing rod 121 into the corresponding fixing hole 0761 for fixing the moving rod 076. The limiting spring 124 can facilitate automatic reset of the moving rod 076, and when the fixing rod 121 is inserted into the fixing hole 0761, the limiting spring 124 can limit the fixing rod 121, reducing the possibility of it being ejected outward due to the impact of carbon dioxide, and improving the stability of fixing the moving rod 076. The pull ring 125 facilitates the movement of the fixing rod 121 by the operator, improving the convenience of use.

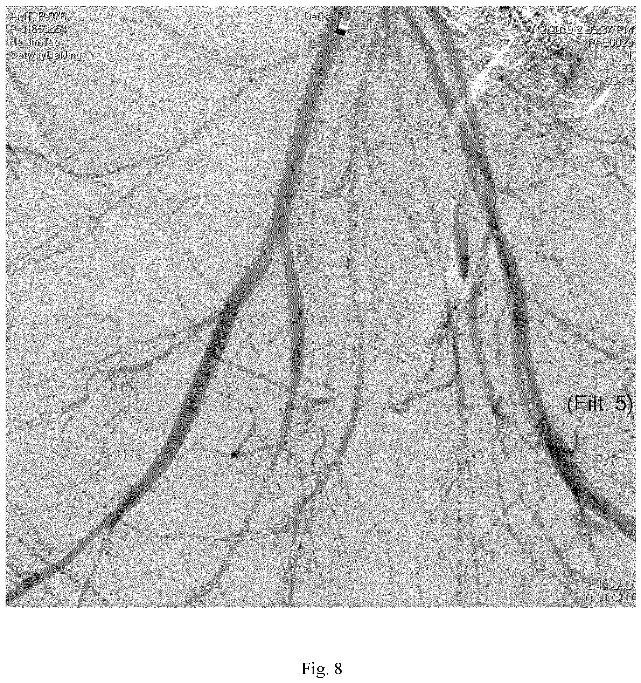

Referring to Fig. 8, when the femoral artery of a human body needs to be radiographed, carbon dioxide is injected into the vessels, and after injection, the femoral artery will be shown in the corresponding angiographic image.

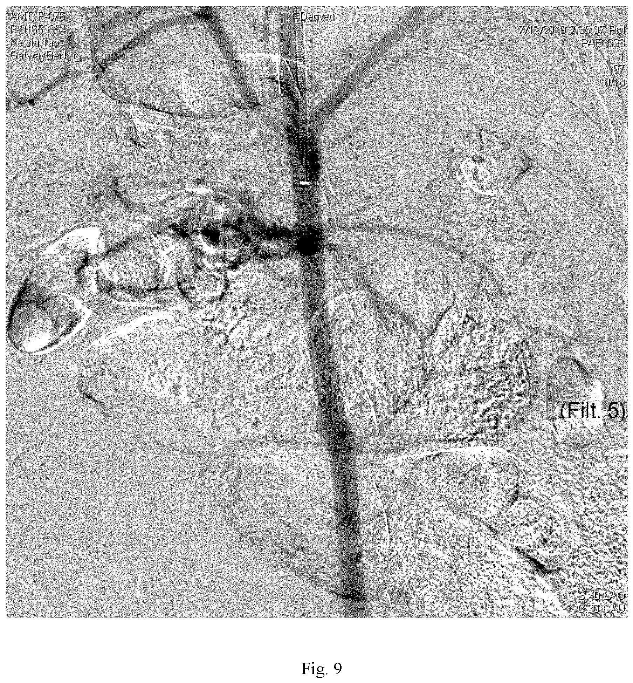

Referring to Fig. 9, when the aorta of a human body needs to be radiographed, carbon dioxide is injected into the vessels, and after injection, the aorta will be shown in the corresponding angiographic image.

This particular embodiment is only an explanation of this application and is not a limitation of it. Those skilled in the art area can make non-inventive modifications to this embodiment based on the description herein, but all fall within the scope of protection of the patent law as long as they are within the range defined by the claims of this application.

## Claims

1. A portable carbon dioxide gas injector, **characterized in that**, the portable carbon dioxide gas injector comprises: an injecting tube (01), one end of which is connected with a passage tube (02), and an injecting valve (03) provided at an end away from the injecting tube (01), of the passage tube (02), an input assembly (04) connected to an end away from the passage tube (02), of the injecting valve (03), a connector (051) connected to an end away from the injecting valve (03), of the input assembly (04), a gas cylinder (052) connected to a side of the connector (051), a piston (011) arranged inside the injecting tube (01) and adapted to the injecting tube (01), a spring (06) arranged on a side away from the passage tube (02), of the piston (11) and configured to drive the piston (011) to move, and a regulating assembly (07) arranged on the side away from the passage tube (02), of the piston (011) and configured to regulate a gas storage volume.

2. The portable carbon dioxide gas injector according to claim 1, wherein one end of the spring (06) is fixedly connected with the piston (011), and the other end of the spring (06) is fixedly connected with an inner wall of the injecting tube (01).

3. The portable carbon dioxide gas injector according to claim 1, wherein the regulating assembly (07) comprises a regulating rod (071) passing through the injecting tube (01), the regulating rod (071) is threaded with the injecting tube (01); a gasket (073) is fixedly arranged at one end close to the piston (011), of the regulating rod (071).

4. The portable carbon dioxide gas injector according to claim 1, wherein the connector (051) is provided with a regulating knob (053) configured to regulate gas pressure input into the injecting tube (01).

5. The portable carbon dioxide gas injector according to claim 4, wherein the connector (051) is provided with a pressure gauge (0511) configured to display gas pressure inside the gas cylinder (052).

6. The portable carbon dioxide gas injector according to claim 1, wherein the input assembly (04) is provided with a filter (08) configured to filter gas injected into the injecting tube (01).

7. The portable carbon dioxide gas injector according to claim 1,wherein the regulating assembly (07) further comprises a moving plate (074) adapted to an inner wall of the injecting tube (01); one end of the spring (06) is fixedly connected with the piston (011), and the other end of the spring (06) is fixedly connected with the moving plate (074); an end face close to the piston (011), of the moving plate (074) is connected to a docking block (075), and an end face away from the piston (011), of the moving plate (074) is connected to a moving rod (076); the injecting tube (01) is provided with a fixing component (12) configured to fix the moving rod (076).

8. The portable carbon dioxide gas injector according to claim 7, wherein the fixing component (12) comprises a fixing rod (121) of which an axis is perpendicular to an axis of the moving rod (076); a plurality of fixing holes (0761) are provided along an extension direction of the moving rod (076); one side of the fixing rod (121) is provided with a fixing box (122) that is fixedly connected with the injecting tube (01), and an end face close to the fixing rod (121), of the fixing box (122) is set to open; a moving block (123) that is fitted with the fixing box (122) is provided inside the fixing box (122), and the moving block (123) is slidably connected to the fixing box (122); the fixing rod (121) passes through the moving block (123) and is fixedly connected to the moving block (123).

9. The portable carbon dioxide gas injector according to claim 8, wherein a limiting spring (24) is sleeved on the fixing rod (121); one end of the limiting spring (124) is fixedly connected to an inner wall away from the moving rod (076), of the fixing box (122), and the other end of the limiting spring (124) is fixedly connected to the moving block (123).

10. The portable carbon dioxide gas injector according to claim 1, wherein the gas cylinder (052) is detachably connected to the connector (051); one end of the injecting valve (03) is connected with a Luer connector (09), an end away from the injecting valve (03), of the Luer connector (09) is provided with a check valve (10); a protective sheath (11) configured to protect the injecting tube (01) is sleeved on an outside of the injecting tube (01).
